# EUROPEAN PATENT SPECIFICATION

(11) **EP 2 813 838 B1**
(45) Date of publication and mention of the grant of the patent: **19.10.2016**
(21) Application number: 12868004.8
(22) Date of filing: 13.12.2012
(51) Int. Cl.: G01N 17/00, G05D 27/02

(54) **ABNORMALITY DETECTION APPARATUS AND ENVIRONMETAL TEST APPARATUS PROVIDED WITH SAME**
FEHLERERKENNUNGSVORRICHTUNG UND UMGEBUNGSTESTVORRICHTUNG DAMIT
APPAREIL DE DÉTECTION D'ANOMALIE ET APPAREIL DE TEST ENVIRONNEMENTAL LE COMPORTANT

(30) Priority: 07.02.2012 JP 2012023712
(43) Date of publication of application: 17.12.2014
(73) Proprietor: Nagano Science Co., Ltd., Takatsuki-shi Osaka 569-1106 (JP)
(72) Inventor: KOBAYASHI, Yuichiro, Takatsuki-shi Osaka 569-1106 (JP); YAMAMOTO, Yusuke, Takatsuki-shi Osaka 569-1106 (JP)
(74) Representative: Herrmann, Uwe
(86) International application number: PCT/JP2012/007990
(87) International publication number: WO 2013/118228

(56) References cited:
- EP-A1- 2 309 247
- EP-A1- 2 351 972
- JP-A- 2001 124 458
- JP-A- 2003 050 061
- JP-A- 2007 218 457
- US-A- 6 079 483

## Description

### TECHNICAL FIELD

The present invention relates to anomaly detectors and environmental testers including the same.

### BACKGROUND ART

For example, an environmental tester including a thermostat-humidistat container (chamber) has been used for stability tests of pharmaceuticals, or the like in order to test the performance of a product under a predetermined temperature and a predetermined humidity. In the environmental tester, a temperature sensor and a humidity sensor are provided in a test chamber of a thermostat-humidistat container surrounded by adiabatic walls, and an air conditioner including a refrigerator, a humidifier, and a heater is controlled based on values measured by these sensors. This allows circulation of air between the test chamber and the air conditioner so that the temperature and the humidity in the test chamber are kept to target temperature and target humidity, respectively (see, for example, Patent Document 1).

An environmental test chamber comprising a refrigerator, a heater and a humidifier is disclosed in Patent Document 2.

### CITATION LIST

### PATENT DOCUMENT

PATENT DOCUMENT 1: Japanese Patent Unexamined Publication No. H07-140061
PATENT DOCUMENT 2 : EP2351972.

### SUMMARY OF THE INVENTION

### TECHNICAL PROBLEM

Incidentally, in a conventional environmental tester, when problems such as leakage of a refrigerant gas and frosting in the refrigerator arise, the temperature and the humidity in the test chamber can no longer be controlled.

Specifically, in the conventional environmental tester, the cooling capacity of the refrigerator is kept constant, and the amounts of electric power supplied to the humidifier and the heater are adjusted, thereby keeping the temperature and the humidity in the test chamber to the target temperature and the target humidity, respectively. Therefore, when the cooling capacity is reduced due to problems such as leakage of a refrigerant gas and frosting in the refrigerator, the capacities of the humidifier and the heater are also reduced along with the reduction in cooling capacity. When the capacities are reduced to or below certain threshold values, the temperature and the humidity in the test chamber can no longer be controlled.

In this regard, temperature sensors may be arranged in a plurality of positions in a refrigerant circuit of the air conditioner, and results of determination by the temperature sensors may be combined to detect a reduction in cooling capacity of the refrigerator before the temperature and the humidity in the test chamber can no longer be controlled. However, arranging the plurality of temperature sensors in the refrigerant circuit increases costs, and depending on positions where the temperature sensors are arranged, the temperature sensors are influenced by changes of an outdoor temperature, so that the control of combining the results of determination becomes complicated.

In view of the foregoing, it is an object of the present invention to allow easy determination of a reduction in cooling capacity of a refrigeration apparatus with a relatively simple configuration.

### SOLUTION TO THE PROBLEM

The present invention is directed to an anomaly detector configured to be add to an environmental tester including a thermostat-humidistat container including a test chamber, a heating device for heating the test chamber at an amount of heating corresponding to an amount of electric power supplied to the heating device, a humidifying device for humidifying the test chamber at a humidification amount corresponding to an amount of electric power supplied to the humidifying device, and a refrigeration apparatus in which a refrigerant is circulated to cool the test chamber by a refrigeration cycle. The present invention has provided the anomaly detector with the following features.

That is, according to a first aspect of the present invention, a controlling section configured to receive a heating electric power value representing the amount of electric power supplied to the heating device and a humidifying electric power value representing the amount of electric power supplied to the humidifying device is .provided. The controlling section compares the heating electric power value and the humidifying electric power value with predetermined reference electric power values of the heating device and the humidifying device, respectively, and when at least one of the heating electric power value or the humidifying electric power value is less than a corresponding one of the reference electric power values, the controlling section makes a determination that a cooling capacity of the refrigeration apparatus has been reduced, and outputs an anomaly signal indicating the determination.

In the first aspect of the invention, in the controlling section, the heating electric power value and the humidifying electric power value are compared with the predetermined reference electric power values of the heating device and the humidifying device, respectively. When at least one of the heating electric power value or the humidifying electric power value is less than a corresponding one of the reference electric power values, the anomaly signal indicating that the cooling capacity of the refrigeration apparatus has been reduced is output.

With this configuration, a reduction in cooling capacity of the refrigeration apparatus can be easily determined with a relatively simple configuration. Specifically, in the conventional environmental tester, the cooling capacity of the refrigeration apparatus is kept constant, and the amounts of electric power supplied to the heating device and the humidifying device are adjusted, thereby keeping the temperature and the humidity in the test chamber to set temperature and humidity. Therefore, when the cooling capacity is reduced due to problems such as leakage of a refrigerant gas or frosting occurred in the refrigeration apparatus, the capacities of the heating device and the humidifying device are also reduced along with the reduction in cooling capacity. When the capacities are reduced to or below certain threshold values, the temperature and the humidity in the test chamber can no longer be controlled.

In contrast, in the present invention, the heating electric power value and the humidifying electric power value are compared with the respective predetermined reference electric power values, and when at least one of the heating electric power value or the humidifying electric power value is less than a corresponding one of the reference electric power values, it is determined that the cooling capacity of the refrigeration apparatus has been reduced. That is, it is determined that a reduction in cooling capacity due to problems such as leakage of a refrigerant gas or frosting occurred in the refrigeration apparatus reduces the capacities of the heating device and the humidifying device. This allows the refrigeration apparatus to be readily replaced before the temperature and the humidity of the test chamber can no longer be controlled.

Since the anomaly detector according to the present invention can be added to an existing environmental tester by retrofitting, it is not necessary to replace the environmental tester, and a reduction in cooling capacity of the refrigeration apparatus can be detected with low costs.

The controlling section of the first aspect of the present invention includes: a receiver configured to receive determined temperature and humidity values representing a temperature and a humidity determined in the test chamber, set temperature and humidity values representing a preset temperature and a preset humidity of the test chamber, the heating electric power value, and the humidifying electric power value; a stability determining section configured to determine that the temperature and the humidity in the test chamber are in a stable state when the determined temperature and humidity values keep matching the set temperature and humidity values, respectively, for a predetermined period of time; a data memory configured to store stable electric power values of the heating device and the humidifying device in the stable state when the stability determining section determines that the temperature and the humidity in the test chamber are in the stable state; and a comparison and determination section configured to compare the heating electric power value and the humidifying electric power value with predetermined reference electric power values of the heating device and the humidifying device, respectively for the determination, and output the anomaly signal. Each of the reference electric power values is obtained by multiplying a corresponding one of the stable electric power values stored in the data memory by a predetermined anomaly detection coefficient.

The controlling section includes the receiver, the stability determining section, the data memory, and the comparison and determination section. The receiver receives the determined temperature and humidity values representing the temperature and the humidity determined in the test chamber, the set temperature and humidity values representing preset temperature and preset humidity of the test chamber, the heating electric power value, and the humidifying electric power value. The stability determining section determines whether or not the temperature and the humidity in the test chamber are in a stable state based on the determined temperature and humidity values and the set temperature and humidity values. The data memory stores stable electric power amounts of the heating device and the humidifying device in the stable state. The comparison and determination section makes a determination based on comparison of the heating electric power value and the humidifying electric power value with the respective predetermined reference electric power values, and outputs the anomaly signal. Here, each of the reference electric power values is obtained by multiplying a corresponding one of the stable electric power values stored in the data memory by the predetermined anomaly detection coefficient.

With this configuration, values obtained by multiplying the stable electric power values of the heating device and the humidifying device in a stable state by predetermined anomaly detection coefficients are used as the reference electric power values, so that a reduction in cooling capacity of the refrigeration apparatus can be more correctly determined.

According to a second aspect of the present invention, an environmental tester includes: the anomaly detector of the first aspect of the present invention; a thermostat-humidistat container including a test chamber; a heating device configured to heat the test chamber at an amount of heating corresponding to an amount of electric power supplied to the heating device; a humidifying device configured to humidify the test chamber at a humidification amount corresponding to an amount of electric power supplied to the humidifying device; a refrigeration apparatus configured to perform a refrigeration cycle by circulating a refrigerant to cool the test chamber; and a warning device configured to perform predetermined warning operation in response to the anomaly signal output from the anomaly detector.

In the second aspect of the invention, when the anomaly signal is output from the anomaly detector, the warning device performs predetermined warning operation. With this configuration, a reduction in cooling capacity of the refrigeration apparatus can be easily determined through the warning operation. The warning device is composed of, for example, an alarm or a signal, and can warn an operator a reduction in cooling capacity by sounding the alarm or turning on the signal. Another example of the warning operation is displaying a message indicating a reduction in cooling capacity on a display or on an external monitor in a remote place via a network to notify a manager.

### ADVANTAGES OF THE INVENTION

According to the present invention, a reduction in cooling capacity of the refrigeration apparatus can be easily determined by simply comparing the heating electric power value and the humidifying electric power value with respective predetermined reference electric power values, and before the temperature and the humidity in the test chamber can no longer be controlled, the refrigeration apparatus can be readily replaced.

Since the anomaly detector according to the present invention can be added to an existing environmental tester by retrofitting, it is not necessary to replace the environmental tester, and a reduction in cooling capacity of the refrigeration apparatus can be detected with low costs.

### BRIEF DESCRIPTION OF THE DRAWINGS

[FIG. 1] FIG. 1 is a perspective view illustrating a configuration of a thermostat-humidistat container in an environmental tester according to an embodiment of the present invention.
[FIG. 2] FIG. 2 is a perspective view illustrating a configuration of a test chamber of the thermostat-humidistat container in the environmental tester.
[FIG. 3] FIG. 3 is a cross section viewed from a side, for schematically illustrating a configuration of the environmental tester.
[FIG. 4] FIG. 4 is a functional block diagram illustrating an internal configuration of the thermostat-humidistat container in the environmental tester.
[FIG. 5] FIG. 5 is a functional block diagram illustrating an internal configuration of an anomaly detector.
[FIG. 6] FIG. 6 is a flow chart illustrating a process of operation of the anomaly detector.

### DESCRIPTION OF EMBODIMENTS

Embodiments according to the present invention will be described with reference to the drawings. The above-described embodiments have been set forth merely for the purposes of preferred examples in nature, and are not intended to limit the scope, applications, and use of the invention.

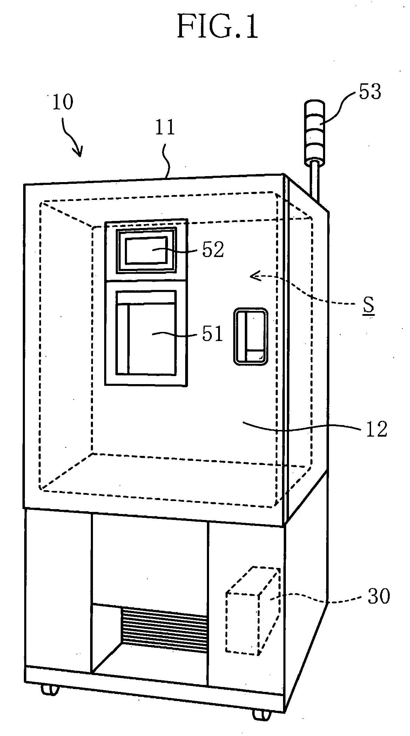

FIG. 1 is a perspective view illustrating a configuration of a thermostat-humidistat container in an environmental tester according to the present embodiment. As illustrated in FIG. 1, a thermostat-humidistat container 11 (chamber) is used for stability tests of pharmaceuticals, for example. For this reason, temperature and humidity in a test chamber S are stably kept within the predetermined ranges, respectively.

The thermostat-humidistat container 11 has a contour in the shape of a substantially rectangular parallelepiped, and a door 12 extending from an upper end to a center section of a front face of the thermostat-humidistat container 11 is attached to the thermostat-humidistat container 11 to be able to open and close. A consol panel 51 for, for example, setting target values for controlling temperature and humidity and a display 52 for displaying the set values, and the like are disposed to be aligned in the vertical direction on a front face of the door 12.

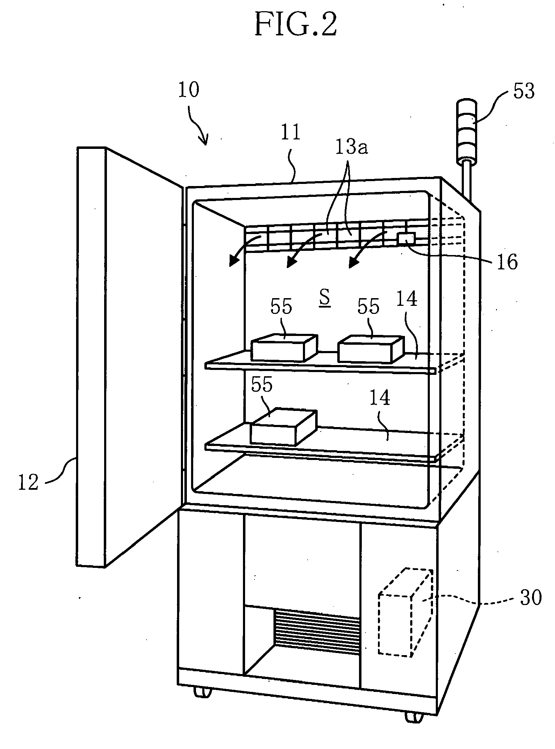

As illustrated in FIG. 2, the door 12 of the thermostat-humidistat container 11 is opened to access to the test chamber S which is in the shape of a substantially rectangular parallelepiped and is formed in the thermostat-humidistat container 11, so that a sample 55 can be placed in the test chamber S, or a sample 55 placed in the test chamber can be taken out.

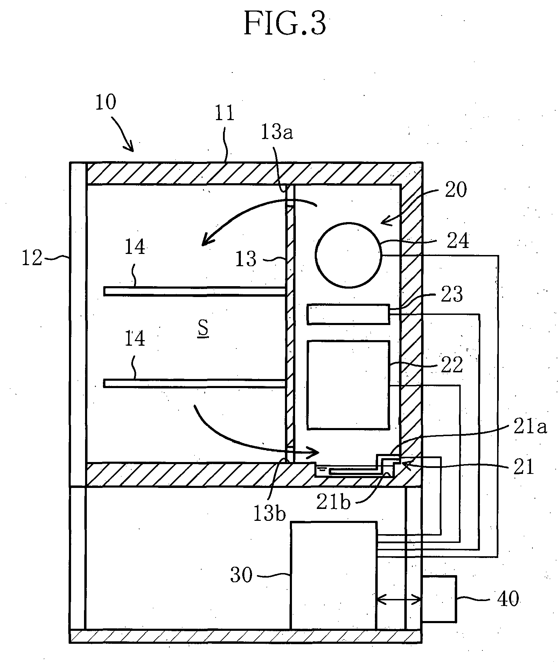

FIG. 3 is a cross section viewed from a side, for schematically illustrating a configuration of the environmental tester. As illustrated in FIG. 3, a lattice-like air outlet 13a is formed in an upper portion of a partitioning wall 13 at the depth of the test chamber S. Through the air outlet 13a, conditioned air of adjusted temperature and humidity is supplied to the test chamber S from an air conditioner 20 by an air blowing fan 24. The conditioned air circulates through the test chamber S to return to the air conditioner 20 via an air inlet 13b at a lower portion of the partitioning wall 13.

In the present embodiment, a configuration in which the air outlet 13a is formed in the upper portion of the partitioning wall 13 at the depth of the test chamber S, and the air inlet 13b is formed in the lower portion of the partitioning wall 13 has been described. However, this configuration is a mere example, and the positions of the air outlet 13a and the air inlet 13b can be suitably determined.

In the test chamber S, two shelf boards 14 are arranged to be aligned in the vertical direction, and samples 55 are placed on the shelf boards 14. The number and the position of the shelf board 14 are not limited to this embodiment, but may be suitably determined.

The thermostat-humidistat container 11 includes a temperature and humidity sensor 16 arranged at the air outlet 13a, and based on values measured by the temperature and humidity sensor 16, the temperature, the relative humidity, and the absolute humidity of air discharged through the air outlet 13a are determined. The arrangement position of the temperature and humidity sensor 16 is not limited to the position near the air outlet 13a, but the temperature and humidity sensor 16 may be arranged near the air inlet. Measured signals from the temperature and humidity sensor 16 are sent to a body controller 30 disposed in a lower portion of the thermostat-humidistat container 11. The body controller 30 controls the air conditioner 20 based on the values measured by the temperature and humidity sensor 16 in such a manner that the temperature and the humidity in the test chamber S match the predetermined temperature and the predetermined humidity, respectively.

Based on control signals output from the body controller 30, the air conditioner 20 suitably adjusts the temperature, the humidity, the flow, etc. of the conditioned air to be supplied to the test chamber S through the air outlet 13a. The air conditioner 20 includes a humidifying device 21, a refrigeration apparatus 22, a heating device 23, and the air blowing fan 24.

The humidifying device 21 includes a heater 21a and a tray 21b in which humidification water is stored. The heater 21a heats the humidification water at the amount of heating corresponding to the amount of electric power supplied to the heater 21a. Water is supplied to the tray 21b from a water supply tank (not shown) provided outside the test chamber S. Based on the control signals output from the body controller 30, the humidifying device 21 operates to evaporate the water stored in the tray 21b by the heater 21a to humidify air to a set humidity. In this way, the humidifying device 21 humidifies the test chamber S at a humidification amount corresponding to the amount of the supplied electric power.

The refrigeration apparatus 22 includes a refrigerant circuit for performing a refrigeration cycle by circulating a refrigerant. Based on the control signals output from the body controller 30, the refrigeration apparatus 22 operates to reduce circulated air to a dew point temperature computed based on a set temperature and the set humidity. This ensures the amount of moisture necessary at the set temperature and the set humidity.

Based on the control signals output from the body controller 30, the heating device 23 operates to increase the temperature of the air cooled by the refrigeration apparatus 22 to the set temperature. In this way, the heating device 23 heats the test chamber S at the amount of heating corresponding to the electric power supplied to the heating device 23.

The air blowing fan 24 operates to circulate the conditioned air adjusted by the humidifying device 21, the refrigeration apparatus 22, and the heating device 23 through the test chamber S. In this way, the air blowing fan 24 circulates the air in the test chamber S, the humidifying device 21, the refrigeration apparatus 22, and the heating device 23 are operated as necessary, and feedback control is performed by determining the temperature and the humidity in the test chamber S by the temperature and humidity sensor 16, thereby stably keeping the temperature and the humidity in the test chamber S within the predetermined ranges.

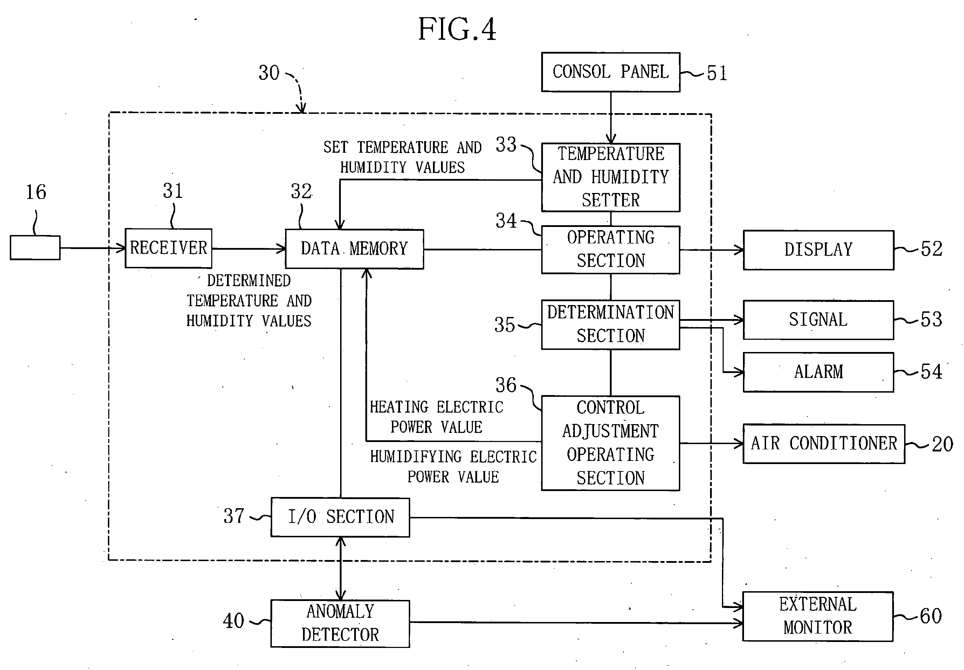

FIG. 4 is a functional block diagram illustrating an internal configuration of the thermostat-humidistat container in the environmental tester. As illustrated in FIG. 4, the body controller 30 includes a receiver 31 for receiving determined temperature and humidity values representing the temperature and the humidity determined by the temperature and humidity sensor 16, and a data memory 32 for storing signals received by the receiver 31.

The body controller 30 further includes a temperature and humidity setter 33 and an operating section 34. The temperature and humidity setter 33 sets target values, i.e., set temperature and humidity values, for controlling the temperature and the humidity in the test chamber S based on signals from the consol panel 51, through which an operator sets the target temperature and humidity. The operating section 34 receives signals from the temperature and humidity setter 33 and the data memory 32, and carries out various operations for controlling the air conditioner 20 as described below. Where necessary, the results of the operations are shown on the display 52 provided on the door 12 of the thermostat-humidistat chamber 11. The set temperature and humidity values are stored in the data memory 32.

The operating section 34 computes the differences of the values of the temperature and the humidity measured by the first temperature and humidity sensor 16 from the set values, thereby generating a correction value. A determination section 35 determines the differences of the values measured by the first temperature and humidity sensor 16 from the set values. Based on the determination results, a signal 53 and an alarm 54 serving as warning devices which give an operator a warning are operated.

The body controller 30 includes a control adjustment operating section 36 which carries out an operation for control adjustments of the air conditioner 20. The control adjustment operating section 36 controls the air conditioner 20 based on the determination results of the determination section 35. Here, a heating electric power value and a humidifying electric power value respectively representing the amount of electric power supplied to the heating device 23 and the amount of electric power supplied to the humidifying device 21 are sent to and stored in the data memory 32.

The body controller 30 further includes an I/O section 37 for communication with an anomaly detector 40 and an external monitor 60. A control program (software) has been updated so that the body controller 30 can perform warning operation based on an anomaly signal output from the anomaly detector 40. The external monitor 60, by which a manager in a remote place monitors the operating state of the environmental tester 10, is connected to the I/O section 37 via a network.

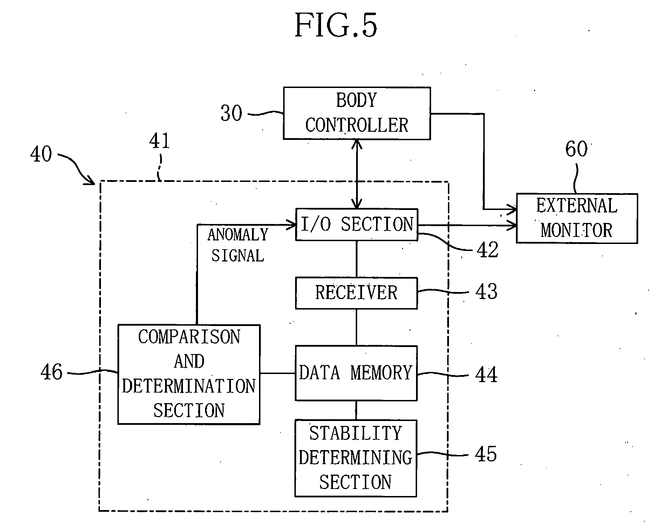

FIG. 5 is a functional block diagram illustrating an internal configuration of the anomaly detector. As illustrated in FIG. 5, the anomaly detector 40 is composed of a controlling section 41 which communicates with the body controller 30 and the external monitor 60.

The controlling section 41 includes an I/O section 42, a receiver 43, a data memory 44, a stability determining section 45, and a comparison and determination section 46. The I/O section 42 is connected to the I/O section 37 of the body controller 30 and to the external monitor 60. The receiver 43 receives various types of data stored in the data memory 32 of the body controller 30. Here, the various types of data include the determined temperature and humidity values representing the temperature and the humidity in the test chamber S which are determined by the temperature and humidity sensor 16, the set temperature and humidity values representing the temperature and the humidity of the test chamber S which are preset via the consol panel 51, the heating electric power value representing the amount of electric power supplied to the heating device 23, and the humidifying electric power value representing the amount of electric power supplied to the humidifying device 21.

The data memory 44 stores the various types of data received by the receiver 43. The stability determining section 45 determines that the temperature and the humidity in the test chamber S are in a stable state when the determined temperature and humidity values keep matching the set temperature and humidity values for a predetermined period of time.

Once the stability determining section 45 has determined that the temperature and the humidity are in the stable state, the data memory 44 stores the heating electric power value and the humidifying electric power value respectively of the heating device 23 and the humidifying device 21 in the stable state as stable electric power values.

The comparison and determination section 46 compares the heating electric power value of the heating device 23 and the humidifying electric power value of the humidifying device 21 with respective predetermined reference electric power values. When at least one of the heating electric power value or the humidifying electric power value is less than a corresponding one of the reference electric power values, the comparison and determination section 46 outputs an anomaly signal representing that the cooling capacity of the refrigeration apparatus 22 has been reduced. Here, each of the reference electric power values is obtained by multiplying a corresponding one of the stable electric power values of the heating device 23 and the humidifying device 21 which are stored in the data memory 44 by a predetermined anomaly detection coefficient.

The anomaly signal output from the comparison and determination section 46 of the anomaly detector 40 is input to the body controller 30 and the external monitor 60 via the I/O section 42. Based on the anomaly signal, the body controller 30 operates the signal 53 and the alarm 54 to warn an operator. The warning operation may be displaying, on the display 52, a message indicating that the cooling capacity of the refrigeration apparatus 22 has been reduced, or may be displaying the message on the external monitor 60 in a remote place to notify a manager via a network.

### -Operating Method-

Next, a method for operating the environmental tester 10 will be described. The door 12 of the thermostat-humidistat container 11 is opened to place a sample 55 of the environmental test on the shelf board 14 disposed in the test chamber S.

Set temperature and humidity values, i.e., target values, of the temperature and the humidity in the test chamber S are input via the consol panel 51 disposed on the front face of the door 12. Here, an operator can see the target values displayed on the display 52 to check whether or not the operation is correct. The set temperature and humidity values are stored in the data memory 32.

During operation of the environmental tester 10, conditioned air of adjusted temperature and humidity is discharged to the test chamber S from the air conditioner 20 through the air outlet 13a. Specifically, in the air conditioner 20, the cooling capacity of the refrigeration apparatus 22 is kept constant, and the amounts of electric power supplied to the heating device 23 and the humidifying device 21 are adjusted. A heating electric power value and a humidifying electric power value respectively representing the amount of electric power supplied to the heating device 23 and the amount of electric power supplied to the humidifying device 21 are stored in the data memory 32.

Air is supplied to the air conditioner 20 through the air inlet disposed in the lower portion of the wall surface at the depth in the test chamber S. The environmental tester 10 is operated such that while the air is circulated in this way between the test chamber S and the air conditioner 20, the conditioned air whose temperature and humidity have been adjusted in the air conditioner 20 is supplied to the test chamber S in such a manner that the temperature and the humidity in the test chamber S match the set temperature and the set humidity which are the input target values.

Determined temperature and humidity values determined by the temperature and humidity sensor 16 disposed near the air outlet 13a in the test chamber S are stored in the data memory 32 via the receiver 31 of the body controller 30. Timing at which the data signals are stored in the data memory 32 occurs in a predetermined cycle.

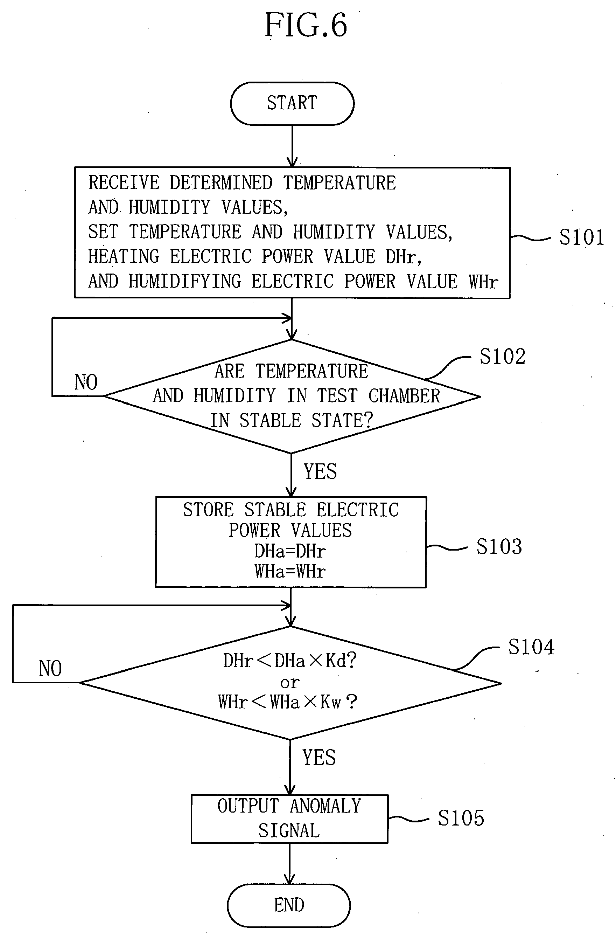

Next, a process of operation of the anomaly detector 40 will be described with reference to the flow chart of FIG. 6. As illustrated in FIG. 6, in step S101, the determined temperature and humidity values, the set temperature and humidity values, the heating electric power value, and the humidifying electric power value which have been stored in the data memory 32 of the body controller 30 are received via the I/O section 42. Then, the process proceeds to step S102. The heating electric power value and the humidifying electric power value are hereinafter denoted by symbols DHr and WHr, respectively.

In step S102, the stability determining section 45 determines whether or not the temperature and the humidity in the test chamber S are in a stable state. Specifically, when the determined temperature and humidity values keep substantially matching the set temperature and humidity values for a predetermined period of time, the temperature and the humidity in the test chamber S are determined to be in the stable state. If "YES" in step S102, the process proceeds to step S103. If "NO" in step S102, the process waits until the stable state is achieved.

In step S103, stable electric power values of the heating device 23 and the humidifying device 21 in the stable state are stored in the data memory 44. Then, the process proceeds to step S104. Specifically, the stable electric power values are stored such that DHa = DHr and WHa = WHr, where DHa represents the stable electric power value of the heating device 23, and WHa represents the stable electric power value of the humidifying device 21.

In step S104, the comparison and determination section 46 compares the heating electric power value and the humidifying electric power value respectively with a predetermined reference electric power value of the heating device 23 and a predetermined reference electric power value of the humidifying device 21 and determine whether or not at least one of the heating electric power value or the humidifying electric power value is less than a corresponding one of the reference electric power value.

Specifically, the reference electric power value of the heating device 23 is obtained by multiplying the stable electric power value DHa of the heating device 23 by an anomaly detection coefficient Kd. The reference electric power value of the humidifying device 21 is obtained by multiplying the stable electric power value WHa of the humidifying device 21 by an anomaly detection coefficient Kw. Here, the anomaly detection coefficients Kd and Kw are values respectively satisfying the relationships 0 < Kd < 1 and 0 < Kw < 1, and are each, for example, 0.5.

The comparison and determination section 46 determines whether or not at least one of the conditions DHr < DHa × Kd or WHr < WHa × Kw is satisfied. If "YES" in step S104, it is determined that the cooling capacity of the refrigeration apparatus 22 has been reduced, and the process proceeds to step S105. If "NO" in step S104, the process waits until the condition is satisfied.

In step S105, an anomaly signal indicating that the cooling capacity of the refrigeration apparatus 22 has been reduced is output, and the process ends. The anomaly signal output from the comparison and determination section 46 of the anomaly detector 40 is sent to the body controller 30 of the environmental tester 10 and the external monitor 60 via the I/O section 42.

Based on the anomaly signal, the body controller 30 operates the signal 53 and the alarm 54 to warn an operator. A message indicating a reduction in cooling capacity of the refrigeration apparatus 22 may be displayed on the display 52, or may be displayed on the external monitor 60 in a remote place via a network to notify a manager.

As described above, in the anomaly detector 40 according to the present embodiment, the value of heating electric power supplied to the heating device 23 and the value of humidifying electric power supplied to the humidifying device 21 are compared with the respective reference electric power values obtained based on stable electric power amounts at the time of the heating device 23 and the humidifying device 21 being in a stable state. When at least one of the value of the heating electric power or the value of the humidifying electric power is less than a corresponding one of the reference electric power values, an anomaly signal indicating that the cooling capacity of the refrigeration apparatus 22 has been reduced is output, so that the reduction in cooling capacity of the refrigeration apparatus 22 can be easily determined, and the refrigeration apparatus 22 can be readily replaced.

Since the anomaly detector 40 according to the present embodiment can be added to the existing environmental tester 10 by retrofitting, it is not necessary to replace the environmental tester 10, and a reduction in cooling capacity of the refrigeration apparatus 22 can be detected with low costs.

### «Other Embodiments»

The embodiment may have the following configuration.

In the above embodiment, the anomaly detector 40 is a device separated from the body controller 30 so that the anomaly detector 40 can be added to the existing environmental tester 10 by retrofitting. However, the function of the controlling section 41 of the anomaly detector 40 may be included in the body controller 30.

In the above embodiment, a configuration in which the thermostat-humidistat container 11 includes the test chamber S provided therein has been described. However, the size of the test chamber S is not particularly limited. That is, a configuration in which a thermostat-humidistat room includes a test chamber S as a room having a size allowing the entrance of workers is also within the scope of the invention.

### INDUSTRIAL APPLICABILITY

As described above, the present invention provides the highly practical advantage that a reduction in cooling capacity of the refrigeration apparatus can be determined with a relatively simple configuration, and is thus very useful and has a wide industrial applicability.

### DESCRIPTION OF REFERENCE CHARACTERS

- 10: Environmental Tester
- 11: Thermostat-Humidistat Container
- 21: Humidifying Device
- 22: Refrigeration Apparatus
- 23: Heating Device
- 40: Anomaly Detector
- 41: Controlling Section
- 43: Receiver
- 44: Data Memory
- 45: Stability Determining Section
- 46: Comparison and Determination Section
- 53: Signal (Warning Device)
- 54: Alarm (Warning Device)
- 60: External Monitor (Warning Device)
- S: Test Chamber

## Claims

1. An anomaly detector (40) configured to be add to an environmental tester (10) including
a thermostat-humidistat container (11) including a test chamber (S),
a heating device (23) for heating the test chamber (S) at an amount of heating corresponding to an amount of electric power supplied to the heating device (23),
a humidifying device (21) for humidifying the test chamber (S) at a humidification amount corresponding to an amount of electric power supplied to the humidifying device (21), and
a refrigeration apparatus (22) in which a refrigerant is circulated to cool the test chamber (S) by a refrigeration cycle,
the anomaly detector (40) comprising:
a controlling section (41) configured to receive a heating electric power value representing the amount of electric power supplied to the heating device (23) and a humidifying electric power value representing the amount of electric power supplied to the humidifying device (21), wherein
the controlling section (41) compares the heating electric power value and the humidifying electric power value with predetermined reference electric power values of the heating device (23) and the humidifying device (21), respectively, and when at least one of the heating electric power value or the humidifying electric power value is less than a corresponding one of the reference electric power values, the controlling section (41) makes a determination that a cooling capacity of the refrigeration apparatus (22) has been reduced, and outputs an anomaly signal indicating the determination, wherein
the controlling section (41) includes
a receiver (43) configured to receive determined temperature and humidity values representing a temperature and a humidity determined in the test chamber (S), set temperature and humidity values representing a preset temperature and a preset humidity of the test chamber (S), the heating electric power value, and the humidifying electric power value,
a stability determining section (45) configured to determine that the temperature and the humidity in the test chamber (S) are in a stable state when the determined temperature and humidity values keep matching the set temperature and humidity values, respectively, for a predetermined period of time,
a data memory (44) configured to store stable electric power values of the heating device (23) and the humidifying device (21) in the stable state when the stability determining section (45) determines that the temperature and the humidity in the test chamber (S) are in the stable state, and
a comparison and determination section (46) configured to compare the heating electric power value and the humidifying electric power value with predetermined reference electric power values of the heating device (23) and the humidifying device (21), respectively for the determination, and output the anomaly signal, wherein
each of the reference electric power values is obtained by multiplying a corresponding one of the stable electric power values stored in the data memory (44) by a predetermined anomaly detection coefficient.

2. An environmental tester (10) comprising:
the anomaly detector (40) of claim 1;
a thermostat-humidistat container (11) including a test chamber (S);
a heating device (23) configured to heat the test chamber (S) at an amount of heating corresponding to an amount of electric power supplied to the heating device (23);
a humidifying device (21) configured to humidify the test chamber (S) at a humidification amount corresponding to an amount of electric power supplied to the humidifying device (21);
a refrigeration apparatus (22) configured to perform a refrigeration cycle by circulating a refrigerant to cool the test chamber (S); and
a warning device configured to perform predetermined warning operation in response to the anomaly signal output from the anomaly detector (40).

## Patentansprüche

1. Anomaliedetektor (40), der an ein Umweltprüfgerät (10) angebaut werden kann, umfassend:
einen Thermostat/Humidistat-Behälter (11) mit einer Prüfkammer (S),
eine Heizvorrichtung (23) zum Heizen der Prüfkammer (S) auf einen Betrag der Erwärmung, der einem Betrag der der Heizvorrichtung (23) zugeführten elektrischen Energie entspricht,
eine Befeuchtungsvorrichtung (21) zum Befeuchten der Prüfkammer (S) auf einen Betrag der Befeuchtung, der einem Betrag der der Befeuchtungsvorrichtung (21) zugeführten elektrischen Energie entspricht, und
eine Kühlvorrichtung (22), in der ein Kühlmittel umgewälzt wird, um die Prüfkammer (S) durch einen Kühlzyklus zu kühlen,
wobei der Anomaliedetektor (40) Folgendes umfasst:
einen Steuerabschnitt (41), der dazu ausgelegt ist, einen Wert der elektrischen Heizenergie, der den Betrag der der Heizvorrichtung (22) zugeführten elektrischen Energie repräsentiert, und einen Wert der elektrischen Befeuchtungsenergie, der den Betrag der der Befeuchtungsvorrichtung (21) zugeführten elektrischen Energie repräsentiert, zu empfangen, wobei
der Steuerabschnitt (41) den Wert der elektrischen Heizenergie und den Wert der elektrischen Befeuchtungsenergie mit vorbestimmten Referenzwerten für die elektrische Energie der Heizvorrichtung (23) bzw. der Befeuchtungsvorrichtung (21) vergleicht, und wenn mindestens einer von dem Wert der elektrischen Heizenergie oder dem Wert der elektrischen Befeuchtungsenergie kleiner ist als ein entsprechender Wert der Referenzwerte für die elektrische Energie, stellt der Steuerabschnitt (41) fest, dass eine Kühlleistung der Kühlvorrichtung (22) verringert wurde, und gibt ein Anomaliesignal aus, das die Feststellung anzeigt, wobei
der Steuerabschnitt (41) Folgendes umfasst:
einen Empfänger (43), der dazu ausgelegt ist, ermittelte Temperatur- und Feuchtigkeitswerte, die eine in der Prüfkammer (S) ermittelte Temperatur und Feuchtigkeit repräsentieren, eingestellte Temperatur- und Feuchtigkeitswerte, die eine voreingestellte Temperatur und eine voreingestellte Feuchtigkeit der Prüfkammer (S) repräsentieren, den Wert der elektrischen Heizenergie und den Wert der elektrischen Befeuchtungsenergie zu empfangen,
einen Stabilitätsermittlungsabschnitt (45), der dazu ausgelegt ist, festzustellen, dass sich die Temperatur und die Feuchtigkeit in der Prüfkammer (S) in einem stabilen Zustand befinden, wenn die ermittelten Temperatur- und Feuchtigkeitswerte weiterhin den eingestellten Temperatur- bzw. Feuchtigkeitswerten für einen vorbestimmten Zeitraum entsprechen,
einen Datenspeicher (44), der dazu ausgelegt ist, stabile Werte für die elektrische Energie der Heizvorrichtung (23) und der Befeuchtungsvorrichtung (21) in dem stabilen Zustand zu speichern, wenn der Stabilitätsermittlungsabschnitt (45) feststellt, dass sich die Temperatur und die Feuchtigkeit in der Prüfkammer (S) in dem stabilen Zustand befinden, und
einen Vergleichs- und Ermittlungsabschnitt (46), der dazu ausgelegt ist, den Wert der elektrischen Heizenergie und den Wert der elektrischen Befeuchtungsenergie mit vorbestimmten Referenzwerten für die elektrische Energie der Heizvorrichtung (23) bzw. der Befeuchtungsvorrichtung (21) für die Ermittlung zu vergleichen und das Anomaliesignal auszugeben, wobei
jeder der Referenzwerte für die elektrische Energie dadurch erhalten wird, dass ein entsprechender, in dem Datenspeicher (44) gespeicherter Wert der stabilen Werte für die elektrische Energie mit einem vorbestimmten Anomalieerkennungskoeffizienten multipliziert wird.

2. Umweltprüfgerät (10), umfassend:
den Anomaliedetektor (40) nach Anspruch 1;
einen Thermostat/Humidistat-Behälter (11) mit einer Prüfkammer (S);
eine Heizvorrichtung (23), die dazu ausgelegt ist, die Prüfkammer (S) auf einen Betrag der Erwärmung zu heizen, der einem Betrag der der Heizvorrichtung (23) zugeführten elektrischen Energie entspricht;
eine Befeuchtungsvorrichtung (21), die dazu ausgelegt ist, die Prüfkammer (S) auf einen Befeuchtungsbetrag zu befeuchten, der einem Betrag der der Befeuchtungsvorrichtung (21) zugeführten elektrischen Energie entspricht;
eine Kühlvorrichtung (22), die dazu ausgelegt ist, einen Kühlzyklus durch Umwälzen eines Kühlmittels zum Kühlen der Prüfkammer (S) durchzuführen; und
eine Warnvorrichtung, die dazu ausgelegt ist, einen vorbestimmten Warnvorgang in Reaktion auf das von dem Anomaliedetektor (40) ausgegebene Anomaliesignal durchzuführen.

## Revendications

1. Détecteur d'anomalie (40) configuré pour être ajouté à un testeur environnemental (10), comprenant
un récipient de thermostat/humidistat (11) comprenant une chambre de test (S),
un dispositif de chauffage (23) pour chauffer la chambre de test (S) sur une quantité de chauffage correspondant à une quantité d'énergie électrique fournie au dispositif de chauffage (23),
un dispositif d'humidification (21) pour humidifier la chambre de test (S) sur une quantité d'humidification correspondant à une quantité d'énergie électrique fournie au dispositif d'humidification (21), et
un appareil de réfrigération (22), dans lequel on fait circuler un réfrigérant pour refroidir la chambre de test (S) par un cycle de réfrigération,
le détecteur d'anomalie (40) comportant :
une section de commande (41) configurée pour recevoir une valeur de l'énergie de chauffage électrique représentant la quantité d'énergie électrique fournie au dispositif de chauffage (23) et une valeur de l'énergie d'humidification électrique représentant la quantité d'énergie électrique fournie au dispositif d'humidification (21), dans laquelle
la section de commande (41) compare la valeur de l'énergie de chauffage électrique et la valeur de l'énergie d'humidification électrique à des valeurs de référence de l'énergie électrique du dispositif de chauffage (23) et du dispositif d'humidification (21), respectivement, et quand au moins une de la valeur de l'énergie de chauffage électrique ou la valeur de l'énergie d'humidification électrique est inférieure à une valeur correspondante des valeurs de référence de l'énergie électrique, la section de commande (41) fait une détermination que la capacité de refroidissement de l'appareil de réfrigération (22) a été réduite, et émet un signal d'anomalie indiquant la détermination, dans laquelle
la section de commande (41) comprend
un récepteur (43) configuré pour recevoir des valeurs de température et d'humidité déterminées représentant une température et une humidité déterminée dans la chambre de test (S), des valeurs de température et d'humidité réglées représentant une température préréglée et une humidité préréglée de la chambre de test (S), la valeur de l'énergie de chauffage électrique et la valeur de l'énergie d'humidification électrique,
une section de détermination de stabilité (45) configurée pour déterminer que la température et l'humidité dans la chambre de test (S) sont dans un état stable, quand les valeurs de température et d'humidité déterminées continuent de correspondre aux valeurs de température et d'humidité réglées, respectivement, pendant un temps prédéterminé,
une mémoire de données (44) configurée pour stocker des valeurs stables de l'énergie électrique du dispositif de chauffage (23) et du dispositif d'humidification (21) dans un état stable, quand la section de détermination de stabilité (45) détermine que la température et l'humidité dans la chambre de test (S) sont dans un état stable, et
une section de comparaison et de détermination (46) configurée pour comparer la valeur de l'énergie de chauffage électrique et la valeur de l'énergie d'humidification électrique à des valeurs de référence prédéterminées de l'énergie électrique du dispositif de chauffage (23) et du dispositif d'humidification (21), respectivement, pour la détermination, et pour émettre le signal d'anomalie, dans laquelle
chacune des valeurs de référence de l'énergie électrique est obtenue en multipliant une valeur correspondante des valeurs stables de l'énergie électrique stockées dans la mémoire de données (44) par un coefficient de détection d'anomalie prédéterminé.

2. Testeur environnemental (10), comportant :
le détecteur d'anomalie (40) selon la revendication 1 ;
un récipient de thermostat/humidistat (11) comprenant une chambre de test (S) ;
un dispositif de chauffage (23) configuré pour chauffer la chambre de test (S) sur une quantité de chauffage correspondant à une quantité d'énergie électrique fournie au dispositif de chauffage (23) ;
un dispositif d'humidification (21) configuré pour humidifier la chambre de test (S) sur une quantité d'humidification correspondant à une quantité d'énergie électrique fournie au dispositif d'humidification (21) ;
un appareil de réfrigération (22) configuré pour effectuer un cycle de réfrigération en circulant un réfrigérant pour refroidir la chambre de test (S) ; et
un dispositif d'avertissement configuré pour effectuer une opération d'avertissement prédéterminée en réponse au signal d'anomalie émis par le détecteur d'anomalie (40).
